(19) Europäisches Patentamt

European Patent Office

Office européen des brevets

(11) **EP 4 052 789 A1**

(12) **EUROPEAN PATENT APPLICATION**

(43) Date of publication:
**07.09.2022 Bulletin 2022/36**

(21) Application number: **21159856.0**

(22) Date of filing: **01.03.2021**

(51) International Patent Classification (IPC):
**B01J 23/75** (2006.01)        **C07C 45/49** (2006.01)
**C07C 47/02** (2006.01)        **B01J 27/20** (2006.01)

(52) Cooperative Patent Classification (CPC):
(C-Sets available)
**B01J 27/00; C07C 45/49**                    (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Evonik Operations GmbH
45128 Essen (DE)**

(72) Inventors:
• **WOLF, Dorit
  61440 Oberursel (DE)**

• **JANTKE, Ralf
  63776 Mömbris (DE)**
• **WEIDLICH, Stephan
  63457 Hanau (DE)**

(74) Representative: **Evonik Patent Association
c/o Evonik Industries AG
IP Management
Bau 1042A/PB 15
Paul-Baumann-Straße 1
45772 Marl (DE)**

(54) **PROCESS FOR PRODUCING PROPANAL FROM METHANOL AND SYNGAS USING HETEROGENEOUS CATALYSTS**

(57)    The present invention relates to processes for the selective production of propanal from methanol, carbon monoxide and hydrogen, using heterogeneous catalysts comprising one or more transition metals selected from Co, Ni, Cu, Fe, Mn, Mo, W, Ru, Re, Rh, and carbon, exhibiting a structure selected from graphitic, carbidic, aromatic or amorphous non-graphitizing.

EP 4 052 789 A1

(52) Cooperative Patent Classification (CPC): (Cont.)

C-Sets
**C07C 45/49, C07C 47/02**

**Description**

**Background**

The present invention relates to processes for the selective production of propanal from methanol, carbon monoxide and hydrogen, using heterogeneous catalysts comprising one or more transition metals selected from Co, Ni, Cu, Fe, Mn, Mo, W, Ru, Re, Rh, and carbon, exhibiting a structure selected from graphitic, carbidic, aromatic or amorphous non-graphitizing.

The present invention addresses value chains of significant commercial relevance, since propanal, further, can be transformed into propylene, e.g., by hydrogenation to 1-propanol, while 1-propanol in turn can be transformed into propene via dehydrogenation. Both processes, i.e. hydrogenation of propanal as well as dehydration of propanol, have been described in the prior art (Jens Klabunde, Chris Bischoff, Anthony J. Papa: Propanols. In: Ullmann's Encyclopedia of Industrial Chemistry. Wiley-VCH Verlag GmbH & Co. KGaA., 25. Mai 2018, S. 5) and (Jochen Forstner, Steffen Unser, Sarah Böhringer: EP3092211A1 *Verfahren zur Herstellung von Propen aus Propanol).* Importantly, the corresponding sequence of reactions (1) formation of propanal from methanol, CO and $H_2$ according to the present invention, (2) hydrogenation of propanal to propanol, and (3) dehydration of propanol to propene, is significantly more selective and less complex than established processes, for converting methanol via intermediate dimethylether into propylene or olefin mixtures.

Further, propanal can be converted into methacrolein with formaldehyde via Mannich-condensation (e.g. in the presence of dimethylamine and acetic acid) (Martin Köstner, Matthias Grömping, Alexander Lygin, Rudolf Burghardt: WO2014170223A1 *"Verfahren zur Herstellung von Methylmethacrylat'),* thus yielding a key intermediate for obtaining methacrylic acid and methyl methacrylate. Accordingly, the present invention also contributes to establishing efficient alternatives to state of the art-processes for producing methacrolein, i.e. in particular gas-phase oxidation of isobutene or tert-butanol, and dehydrogenation of isobutyraldehyde (Koichi NAGAI Toshiaki UI, Sumitomo Chemical Co., Ltd. Basic Chemicals Research Laboratory: R&D Report, "SUMITOMO KAGAKU", vol. 2004-11, Trends and Future of Monomer- MMA Technologies.).

Typically, the heterogenous catalytic process according to the present invention is carried out at temperatures in the range of 125°C to 240°C, and pressures in the range of 30 to 300 bar, with a $CO/H_2$ molar ratio in the range of 0.5 to 1.5.

The heterogeneous catalyst employed for the process according to the present invention comprises one or more transition metals selected from Co, Ni, Cu, Fe, Mn, Mo, W, Ru, Re, Rh, and carbon, exhibiting a structure selected from graphitic, carbidic, aromatic or amorphous non-graphitized.

**The prior art**

Propanal is an important raw material in industrial chemistry, e.g. for the production of polymers, plasticizers, phenol resins, agrochemicals, odorants, pharmaceuticals etc. Large-scale products obtained from propanal include 1-propanol, 1-propylamin, propionic

acid, trimethylolethane, methacrolein and propionitrile.

Propanal is produced industrially mainly by hydroformylation of ethylene. Several hundred thousand tons are produced annually by corresponding processes:

$$CO + H_2 + C_2H_4 \rightarrow CH_3CH_2CHO$$

Additionally, there are several other ways of producing propanal, not all of which, however, are suitable for large scale industrial implementation:

- Oxidation of 1-propanol with a mixture of sulfuric acid and potassium dichromate (C. D. Hurd, R. N. Meinert: Propionaldehyde In: Organic Syntheses. 12, 1932, S. 64, doi: 10.15227/orgsyn.012.0064; Coll. Vol. 2, 1943, S. 541)
- Oxidation of 1-propanol using molecular oxygen as oxidant in the presence of copper or platinum as catalysts (P. Sabatier, J.-B. Sanderens: Dédoublement catalytique des alcools par les métaux divisés: alcools primaires forméniques. In: Compt. Rend. Hebd. 136, 1903, S. 921ff.)
- Isomerization of propene oxide at 300 °C using silicagel as catalysts (C. D. Hurd, R. N. Meinert: Propionaldehyde In: Organic Syntheses. 12, 1932, S. 64, doi: 10.15227/orgsyn.012.0064; Coll. Vol. 2, 1943, S. 541)
- Catalytic hydrogenation of acrolein (P. Sabatier, J.-B. Senderens: Nouvelles méthodes générales d'hydrogénation. In: Ann. phys. chim. 8, 4, 1905, S. 398) Grignard-Reaction of Ethyl magnesium bromide with ethyl formate (C. D. Hurd, R. N. Meinert: Propionaldehyde In: Organic Syntheses. 12, 1932, S. 64, doi: 10.15227/orgsyn.012.0064; Coll. Vol. 2, 1943, S. 541)

Importantly, despite the fact that a heterogeneous process for the direct conversion of syngas and methanol to propanal could potentially impact several significant value chains, no such process has been known in the art until now.

In the context of the present invention the terms "direct conversion" or "directly converting" designate the step of a chemical process, wherein starting materials are converted into products in a reactor or set of reactors equipped with only one type of catalytically active material.

In the context of the present invention syngas or synthesis gas is defined as a gas mixture primarily containing hydrogen and carbon monoxide in different compositions, which may, however, also contain other components like e.g., carbon dioxide, nitrogen or hydrocarbons.

**The present invention**

The present invention provides a process employing heterogeneous catalysis for the direct conversion of syngas and methanol to propanal. The process relies on heterogeneous catalysts comprising one or more transition metals selected from Co, Ni, Cu, Fe, Mn, Mo, W, Ru, Re, Rh, and carbon, exhibiting a structure selected from graphitic, carbidic, aromatic or amorphous non-graphitizing.

As indicated above, combining the process of the present invention with prior art processes for converting propanal into propanol and propene subsequently, the present invention opens up economically attractive routes from syngas/methanol to propene offering significantly higher selectivity and less complexity than established processes for transforming methanol directly or via intermediate steps into propylene or other olefins, like e.g. the various MTO (Methanol to Olefins) and MTP (Methanol to Propene) processes.

Additional options for downstream processing of propanal include conversion to methacrolein via Mannich reaction and further transformation of methacrolein into methyl methacrylate.

In the course of experiments performed in conjunction with the present invention, it was found that heterogeneous catalysts comprising one or more transition metals selected from Co, Ni, Cu, Fe, Mn, Mo, W, Ru, Re, Rh, and carbon, exhibiting a structure selected from graphitic, carbidic, aromatic or amorphous non-graphitizing can be employed for the direct conversion of carbon monoxide, hydrogen and methanol to propanal.

The catalysts employed in the processes of the invention comprise a carbon matrix.

This carbon matrix may exhibit any of the following types of structure: Graphitic, carbidic, aromatic or amorphous non-graphitizing.

In the context of the present invention graphitic carbon is defined as carbon contained in a graphite structure, i.e. sp2 -hybridized carbon contained in planar aromatic structures consisting of more than 10 rings, with more than four of these planar structures bound to each other via van der Waals forces. The structure of graphitic carbon exhibits long-range crystalline order.

In the context of the present invention carbidic carbon is defined as carbon bound to a metal species via ionic, metallic or covalent bond.

In the context of the present invention aromatic carbon is defined as carbon contained in planar ring structures, wherein every atom in the ring structure has an occupied p orbital, which overlaps with p orbitals on either side (completely conjugated) and wherein the ring structure contains an odd number of pairs of pi electrons satisfying Hückel's rule: $(4n+2)$ pi electrons, where n is an integer starting at zero.

In the context of the present invention amorphous non-graphitizing carbon is defined as amorphous carbon, which contains planar aromatic structures with less than four such planar structures bound to each other, wherein cross-linking between these planar structures prevents graphitization. Amorphous non-graphitizing carbon does not exhibit long-range crystalline order.

The one or more transition metals selected from Co, Ni, Cu, Fe, Mn, Mo, W, Ru, Re, Rh can be present on the surface of the carbon matrix, embedded therein or both.

In a preferred embodiment of the present invention the one or more transition metals are selected from Co, Cu, Mn, Fe, Ni.

In another preferred embodiment of the present invention the one or more transition metals are selected from Co, Cu, and Mn.

In another preferred embodiment of the present invention the one or more transition metals are selected from Co, Fe, Ni.

In another preferred embodiment of the present invention the one or more transition metals are selected from Co.

Methods for obtaining such catalysts have been provided in the art, e.g. (Westerhaus, Felix A., et al. "Heterogenized cobalt oxide catalysts for nitroarene reduction by pyrolysis of molecularly defined complexes" Nature Chemistry (2013)), (B. Hasse, J. Gläsel, A.M. Kern, D.Yu. Murzin, B.J.M. Etzol, Catalysis Today, Volume 249, 1 July 2015, Pages 30-37), (Edmond Lam and John H. T. Luong: ACS Catal. 2014, 4, 3393-3410, dx.doi.org/10.1021/cs5008393 | "Carbon Materials as Catalyst Supports and Catalysts in the Transformation of Biomass to Fuels and Chemicals').

In the course of experiments performed in conjunction with the present invention, it was found that heterogeneous catalysts comprising materials exhibiting a high dispersion and uniform coordination of transition metal particles in combination with a high metal content are particularly effective for catalyzing the processes of the invention. The present invention specifically defines structural characteristics of such materials as well as facile processes for their manufacture. Accordingly, in another preferred embodiment of the present invention, the heterogeneous catalyst comprises catalytically active material, comprising grains of non-graphitizing carbon with cobalt nanoparticles dispersed therein, wherein

$d_p$, the average diameter of cobalt nanoparticles in the non- graphitizing carbon grains, is in the range of 1 nm to 20 nm,

D, the average distance between cobalt nanoparticles in the non- graphitizing carbon grains, is in the range of 2 nm to 150 nm, and

$\omega$, the combined total mass fraction of metal in the non- graphitizing carbon grains, is in the range of 30 wt% to 70 wt% of the total mass of the non- graphitizing carbon grains,

wherein $d_p$ and D are measured by TGZ-TEM as described herein,

and wherein

$d_p$, D and $\omega$ conform to the following relation:

$$4.5\, d_p\, /\, \omega > D \geq 0.25\, d_p\, /\, \omega.$$

Material comprising grains of non-graphitizing carbon with cobalt nanoparticles dispersed therein, exhibiting the specific structural characteristics presently disclosed, can be obtained by a process comprising the following steps:

(a) providing an aqueous solution comprising metal precursor and organic carbon source,

wherein the metal precursor comprises one or a combination of more than one organic, at least partially water soluble, salts of cobalt, and

wherein the organic carbon source is one or a combination of more than one di-, tri-, or polycarboxylic acids,

(b) spray drying or freeze drying the aqueous solution of metal precursor and organic carbon source and, thus, obtaining intermediate product P,

(c) thermo-treating intermediate product P at a temperature in the range from 200 °C to 380 °C.

As a result of research underlying the present invention it was found that grains of non-graphitizing carbon with cobalt nanoparticles dispersed therein, exhibiting the specific structural characteristics presently disclosed, can be obtained from aqueous solutions of metal precursors and organic carbon sources by combining

(i) spray drying or freeze drying of the aqueous solution, with
(ii) thermal treatment at moderate temperatures of the intermediate obtained from step (i).

The final product was found to exhibit catalytic activity in the process of the present invention.
In the context of the present invention, any material or substance lowering the activation energy of a chemical reaction without being consumed by the catalyzed reaction itself, is considered to be a catalyst and thus as catalytically active.
It was found that forming aqueous solutions of metal precursors and organic carbon sources in glass beakers and slowly drying these solutions overnight in a drying cabinet did not yield intermediate products that could be transformed into grains of non-graphitizing carbon with cobalt nanoparticles dispersed therein by thermal treatment at moderate temperatures. Specifically, it was found that if the drying process was performed too slowly, significant decomposition of polycarboxylic acids and formation of carbon dioxide started too early, leading to an early loss of oxygen functionalities of the carbon source. An early loss of oxygen functionalities, however, appears to correlate with an agglomeration of metal components and a segregation of metal precursor and carbon source, ultimately yielding an irregular distribution of large size metal clusters within the carbon matrix. Without wanting to be bound by theory, thus, it appears that sufficient availability of oxygen containing functional groups during parts of the drying procedure appears to be essential for fixing metal precursors within the carbon source in a highly dispersed and regular manner.

It was, furthermore, found that thermo-treating intermediate product P at temperatures below 200 °C and above 380 °C did not yield grains of non-graphitizing carbon with cobalt nanoparticles dispersed therein. It was found, in particular, that the proportion of the non-graphitizing carbon phase itself decreased when the temperatures selected for thermo-treating were too high. These phases, however, are putatively related to expedient hydrogen conductivity which, in turn, is beneficial for efficiently catalyzing reactions involving the conversion of hydrogen. If on the other hand, temperatures selected for thermo-treating were too low or the duration of thermo-treating was too short, the level of residual oxygen in the carbon phase obtained was too high and reduction of metal precursors remained incomplete, leading to lowered catalytic activity as a result. It should be noted, in addition, that, in view of the prior art, formation of the non-graphitizing carbon phase under the conditions presently disclosed, may appear to be surprising. However, without wanting to be bound by theory, it is assumed that formation of non-graphitizing carbon under low temperature conditions, as presently disclosed, is facilitated by the presence of high concentrations of metal precursors in a highly dispersed manner in intermediate product P before subsequent thermo-treating.

The process presently disclosed yields non-graphitizing carbon material in granular form. Non-graphitizing carbon can be identified by a person of skill using TEM-analysis (cf. P.W. Albers, Neutron scattering study of the terminating protons in the basic structural units of non-graphitizing and graphitizing carbons, Carbon 109 (2016), 239 - 245, page 241, figure 1c).

Experimental results obtained in conjunction with the present invention indicate that the catalytic activity of material obtained by the process presently disclosed, was particularly high when the content of grains of non-graphitizing carbon exhibiting the specific features of the preferred catalytic material was high.

Typically, 90% of the non-graphitizing carbon grains obtained by the process presently disclosed exhibit moderate size, i.e. diameters between 2 $\mu$m and 200 $\mu$m. It was presently found that, generally, more than 95% of those moderately sized non-graphitizing carbon grains, obtained by the process presently disclosed, contain cobalt nanoparticles dispersed therein that conform to the relation 4.5 $d_p$ / $\omega$ > D $\geq$ 0.25 $d_p$ / $\omega$ (with $d_p$ denoting the average diameter of cobalt nanoparticles in the non-graphitizing carbon grains, D, denoting the average distance between cobalt nanoparticles in the non-graphitizing carbon grains, and $\omega$, denoting the combined total mass fraction of metal in the non-graphitizing carbon grains). The process presently disclosed, typically, yields grains wherein, only the fraction of very small and the fraction of very large grains, i.e. the particle-fractions outside of the moderate size range between 2 $\mu$m and 200 $\mu$m, contain significant portions of grains wherein cobalt nanoparticles do not conform to the relation 4.5 $d_p$ / $\omega$ > D $\geq$ 0.25 $d_p$ / $\omega$. Accordingly, the process presently disclosed, generally, yields materials with a high content of grains containing cobalt nanoparticles, wherein cobalt nanoparticles conform to the relation 4.5 $d_p$ / $\omega$ > D $\geq$ 0.25 $d_p$ / $\omega$. However, materials with lower contents of these grains may be obtained by other processes or dilution with other materials.

Accordingly, in a preferred embodiment the present invention relates to processes for transforming methanol into a product mixture comprising propanal, comprising the step of contacting methanol, CO and $H_2$ with a heterogeneous catalyst comprising catalytically active material, comprising grains of non-graphitizing carbon with cobalt nanoparticles dispersed therein, wherein cobalt nanoparticles in more than 90% of moderately sized non-graphitizing carbon grains, i.e. non-graphitizing carbon grains with a diameter between 2 $\mu$m and 200 $\mu$m conform to the relation 4.5 dp / $\omega$ > D $\geq$ 0.25 dp / $\omega$, and wherein further dp, the average diameter of cobalt nanoparticles in the non-graphitizing carbon grains, is in the range of 1 nm to 20 nm, D, the average distance between cobalt nanoparticles in the non-graphitizing carbon grains, is in the range of 2 nm to 150 nm, and $\omega$, the combined total mass fraction of metal in the non-graphitizing carbon grains, is in the range of 30 wt% to 70 wt% of the total mass of the non-graphitizing carbon grains.

In another preferred embodiment, the present invention relates to processes for transforming methanol into a product mixture comprising propanal, comprising the step of contacting methanol, CO and $H_2$ with a heterogeneous catalyst comprising catalytically active material, comprising grains of non-graphitizing carbon with cobalt nanoparticles dispersed therein, wherein cobalt nanoparticles in more than 95% of moderately sized non-graphitizing carbon grains, i.e. non-graphitizing carbon grains with a diameter between 2 $\mu$m and 200 $\mu$m conform to the relation 4.5 dp / $\omega$ > D $\geq$ 0.25 dp / $\omega$, and wherein further dp, the average diameter of cobalt nanoparticles in the non-graphitizing carbon grains, is in the range of 1 nm to 20 nm, D, the average distance between cobalt nanoparticles in the non-graphitizing carbon grains, is in the range of 2 nm to 150 nm, and $\omega$, the combined total mass fraction of metal in the non-graphitizing carbon grains, is in the range of 30 wt% to 70 wt% of the total mass of the non-graphitizing carbon grains.

The cobalt nanoparticles in the non-graphitizing carbon material presently disclosed are mainly composed of elementary cobalt but may also contain, for example, cobalt oxide and/or dopant metal.

Computer aided analysis of TEM-pictures (TEM = transmission electron microscopy) coupled with Degussa derived TGZ method allows to determine diameters of individual cobalt nanoparticles as well as statistical measures of sets thereof (cf. Parker et al. "The effect of particle size, morphology and support on the formation of palladium hydride in commercial catalysts" Chemical Science, 2019, 10, 480).

In the context of the present invention, the average diameter of cobalt nanoparticles, $d_p$, and the average distance D is determined by the TGZ-TEM method, as described in the following:

1. Sample Preparation

In most cases, the samples to be tested are available as powders.

The powders are usually dispersed in solvents under ultrasonic application. The ultrasonic application breaks down agglomerates into aggregates and the result is an aggregate distribution rather than a mixture of aggregates and agglomerates. A micro pipette is then used to drop a drop onto a film-coated mesh lying on a piece of filter paper. The excess liquid is quickly sucked off through the filter paper so that agglomerate formation is prevented by the drying process. The suspended grains must not be too dense, as the shape and outline of the nanoparticles cannot be clearly seen through contact and overlapping of grains. An optimal dilution must be determined by test experiments with a dilution series.

In general, it can be stated that the type of preparation has hardly any effect on the result of the primary nanoparticle size evaluation.

2. Performance of the test

The individual nanoparticles to be characterized on the basis of the TEM images must be imaged with sufficiently sharp contours.

A distribution of the nanoparticles that is not too dense with few overlaps or particles that are as separated from each other as possible on the TEM images facilitates the measurement on the TGZ3, but does not influence the measurement result.

After examining various image sections of a TEM preparation, suitable areas are selected accordingly. It should be noted that the ratio of small, medium and large nanoparticles for the respective sample is representative and characteristic and no selective preference of small or large particles is given by the operator.

The total number of primary nanoparticles to be measured depends on the scattering range of the primary nanoparticle size: the larger the scattering range, the more particles have to be measured to obtain an adequate statistical statement. For metal catalysts approx. 1500 single particles are measured. For all TGZ analysis a calibrated Hitachi H-7500 field transmission electron microscope operated at 100 keV, equipped with a CCD-Camera was used.

3. Description of the measurement procedure

The measurement procedure is done according to the TGZ3 manual by Carl ZEISS ("Teilchengrößenanalysator (particle size analyser) TGZ3"; Manual Fa. Carl ZEISS).

4. Measurement data processing

A detailed description of the measurement data processing is given in (F. Endter u. H. Gebauer, "Optik (Optics)" 13 (1956), 97) and (K. Seibold und M. Voll, "Distribution function for describing the particle size distribution of Soot and pyrogenic oxides". Chemiker-Zeitung, 102 (1978), Nr. 4, 131-135).

The statistical summary is compiled in the form of a report. A detailed statistical description is given in (Lothar Sachs, "Statistical methods", 5. Auflage, Springer-Verlag, Berlin (1982)).

5. Evaluation and Display of Results

a. Total number of particles (N)
b. Particle size distributions q0(x) and q3(x) evaluated of 1500 isolated nanoparticles per sample
c. Particle diameter $d_n$, mean diameter ($d_n$)

$$d_n = \frac{\sum n_i d_i}{\sum n_i} = \frac{\sum n_i d_i}{n}$$

$n_i$ = number of particles with diameter $d_i$
d. Average distance D on rectangular plane

$$D = \frac{1}{a^2 b^2} \int_0^b dy^* \int_0^b dy \int_0^a dx^* \int_0^a \sqrt{(x^* - x)^2 + (y^* - y)^2} \, dx$$

a, b = length, width of the rectangular plane
x, y, x*,y* = particle coordinates.

The combined total mass fraction of metal, $\omega$, is defined as the fraction of the combined total masses of cobalt and all dopant metals, of the total mass of the material under consideration: $\omega$ = (m(cobalt) + m(dopant metals)) / m(material); with m(cobalt) = total mass of cobalt in elemental form contained in the material in the form of elemental cobalt itself and/or in the form of any compounds of cobalt, m(dopant metals) = combined total mass of all dopant metals in elemental form contained in the material in the form of the elemental dopant metals themselves and/or in the form of any compounds of the dopant metals, and m(material) = total mass of material under consideration.

The combined total mass fraction of metal, $\omega$, can be determined by means of all methods for quantitative elementary analysis, in particular XRF (X-ray fluorescence) and ICP-AES (Inductively coupled plasma atomic emission spectroscopy).

A suitable choice of conditions in the process presently disclosed allows to control the combined total mass fraction of metal, $\omega$, in the material obtained:

Processes providing in step (a), solutions with a high metal content (cobalt and dopant metals combined), yield materials with a higher combined total mass fraction of metal, $\omega$, than processes providing in step (a) solutions with a lower metal content.

Processes with thermo-treating in step (c) at high temperatures in the range from 200 °C to 380 °C yield materials with a higher combined total mass fraction of metal, $\omega$, than processes with thermo-treating in step (c) at lower temperatures.

The process presently disclosed yields granular material. The size of individual particles of this material as well as statistical measures of sets thereof can be determined by means of laser diffraction analysis (e.g. Cilas 1190 Series), well known to persons of skill in this field.

Typically, the process presently disclosed yields granular material exhibiting the following particle size distribution: d10 $\leq$ 5$\mu$m, d50 $\leq$ 40 $\mu$m, d90 $\leq$ 150 $\mu$m.

In view of the fact that material obtained by the process presently disclosed was found to be very suitable for manufacturing shaped catalysts, in a preferred embodiment the present invention relates to processes for transforming methanol into a product mixture comprising propanal, comprising the step of contacting methanol, CO and $H_2$ with a heterogeneous catalyst comprising catalytically active material, comprising grains of non-graphitizing carbon with cobalt nanoparticles dispersed therein, wherein

$d_p$, the average diameter of cobalt nanoparticles in the non-graphitizing carbon grains, is in the range of 1 nm to 20 nm, D, the average distance between cobalt nanoparticles in the non-graphitizing carbon grains, is in the range of 2 nm to 150 nm, and

$\omega$, the combined total mass fraction of metal in the non-graphitizing carbon grains, is in the range of 30 wt% to 70 wt% of the total mass of the non-graphitizing carbon grains,

and wherein

$d_p$, D and $\omega$ conform to the following relation:

$$4.5 \, d_p \, / \, \omega > D \geq 0.25 \, d_p \, / \, \omega,$$

and wherein

the non-graphitizing carbon grains exhibit the following particle size distribution: d10 = 5$\mu$m, d50 = 40 $\mu$m, d90 = 150 $\mu$m.

There may be applications for materials where the presence of Nitrogen is detrimental. Accordingly, in a preferred embodiment, the present invention relates to processes for transforming methanol into a product mixture comprising propanal, comprising the step of contacting methanol, CO and $H_2$ with a heterogeneous catalyst comprising catalytically active material, comprising grains of non-graphitizing carbon with cobalt nanoparticles dispersed therein, wherein

dp, the average diameter of cobalt nanoparticles in the non-graphitizing carbon grains, is in the range of 1 nm to 20 nm, D, the average distance between cobalt nanoparticles in the non-graphitizing carbon grains, is in the range of 2 nm to 150 nm, and

$\omega$, the combined total mass fraction of metal in the non-graphitizing carbon grains, is in the range of 30 wt% to 70 wt% of the total mass of the non-graphitizing carbon grains,

and wherein

dp, D and $\omega$ conform to the following relation:

$$4.5 \, dp \, / \, \omega > D \geq 0.25 \, dp \, / \, \omega,$$

and

wherein the total mass fraction of nitrogen in the non-graphitizing carbon grains in the catalytically active material is less than 1 wt% of the total mass of the non-graphitizing carbon grains.

Experimental results indicate that material with relatively small cobalt nanoparticles may exhibit particularly attractive catalytic properties. Accordingly, in a preferred embodiment, the present invention relates to processes for transforming methanol into a product mixture comprising propanal, comprising the step of contacting methanol, CO and $H_2$ with a heterogeneous catalyst comprising catalytically active material, comprising grains of non-graphitizing carbon with cobalt nanoparticles dispersed therein, wherein

dp, the average diameter of cobalt nanoparticles in the non-graphitizing carbon grains, is in the range of 1 nm to 10 nm,
D, the average distance between cobalt nanoparticles in the non-graphitizing carbon grains, is in the range of 2 nm to 150 nm, and
ω, the combined total mass fraction of metal in the non-graphitizing carbon grains, is in the range of 30 wt% to 70 wt% of the total mass of the non-graphitizing carbon grains,
and wherein
dp, D and ω conform to the following relation:

$$4.5\, dp\, /\, \omega > D \geq 0.25\, dp\, /\, \omega.$$

In a particularly preferred embodiment, the present invention relates to processes for transforming methanol into a product mixture comprising propanal, comprising the step of contacting methanol, CO and $H_2$ with a heterogeneous catalyst comprising catalytically active material, comprising grains of non-graphitizing carbon with cobalt nanoparticles dispersed therein, wherein
dp, the average diameter of cobalt nanoparticles in the non-graphitizing carbon grains, is in the range of 2 nm to 6 nm,
D, the average distance between cobalt nanoparticles in the non-graphitizing carbon grains, is in the range of 2 nm to 150 nm, and
ω, the combined total mass fraction of metal in the non-graphitizing carbon grains, is in the range of 30 wt% to 70 wt% of the total mass of the non-graphitizing carbon grains,
and wherein
dp, D and ω conform to the following relation:

$$4.5\, dp\, /\, \omega > D \geq 0.25\, dp\, /\, \omega.$$

As indicated by experimental results, addition of dopant metals may affect catalytic activity of the catalytically active materials. Accordingly, in a preferred embodiment, the present invention relates to processes for transforming methanol into a product mixture comprising propanal, comprising the step of contacting methanol, CO and $H_2$ with a heterogeneous catalyst comprising catalytically active material, comprising grains of non-graphitizing carbon with cobalt nanoparticles dispersed therein, wherein
dp, the average diameter of cobalt nanoparticles in the non-graphitizing carbon grains, is in the range of 1 nm to 20 nm,
D, the average distance between cobalt nanoparticles in the non-graphitizing carbon grains, is in the range of 2 nm to 150 nm, and
ω, the combined total mass fraction of metal in the non-graphitizing carbon grains, is in the range of 30 wt% to 70 wt% of the total mass of the non-graphitizing carbon grains,
and wherein
dp, D and ω conform to the following relation:

$$4.5\, dp\, /\, \omega > D \geq 0.25\, dp\, /\, \omega,$$

and wherein the heterogeneous catalyst has been doped with dopant metal, and wherein the dopant metal is selected from Mn, Cu or mixtures thereof, and wherein the material exhibits a molar ratio RDM = n(cobalt) : n(dopant metal) in the range of 2 to 15.
In a particularly preferred embodiment, the present invention relates to processes for transforming methanol into a product mixture comprising propanal, comprising the step of contacting methanol, CO and $H_2$ with a heterogeneous catalyst comprising catalytically active material, comprising grains of non-graphitizing carbon with cobalt nanoparticles dispersed therein, wherein
dp, the average diameter of cobalt nanoparticles in the non-graphitizing carbon grains, is in the range of 1 nm to 20 nm,
D, the average distance between cobalt nanoparticles in the non-graphitizing carbon grains, is in the range of 2 nm to 150 nm, and
ω, the combined total mass fraction of metal in the non-graphitizing carbon grains, is in the range of 30 wt% to 70 wt% of the total mass of the non-graphitizing carbon grains,
and wherein
dp, D and ω conform to the following relation:

$$4.5\ dp\ /\ \omega > D \geq 0.25\ dp\ /\ \omega,$$

and wherein the heterogeneous catalyst has been doped with dopant metal, and wherein the dopant metal is selected from Mn, Cu or mixtures thereof, and wherein the material exhibits a molar ratio RDM = n(cobalt) : n(dopant metal) in the range of 4 to 10.

Experimental results indicate that material with a very low content of Copper may exhibit particularly attractive catalytic properties. Accordingly, in a preferred embodiment, the present invention relates to processes for transforming methanol into a product mixture comprising propanal, comprising the step of contacting methanol, CO and $H_2$ with a heterogeneous catalyst comprising catalytically active material, comprising grains of non-graphitizing carbon with cobalt nanoparticles dispersed therein, wherein

dp, the average diameter of cobalt nanoparticles in the non-graphitizing carbon grains, is in the range of 1 nm to 20 nm,
D, the average distance between cobalt nanoparticles in the non-graphitizing carbon grains, is in the range of 2 nm to 150 nm, and
$\omega$ , the combined total mass fraction of metal in the non-graphitizing carbon grains, is in the range of 30 wt% to 70 wt% of the total mass of the non-graphitizing carbon grains,
and wherein
dp, D and $\omega$ conform to the following relation:

$$4.5\ dp\ /\ \omega > D \geq 0.25\ dp\ /\ \omega,$$

and wherein the total mass fraction of Cu is less than $10^{-4}$ wt% of the total mass of the non-graphitizing carbon grains.

Catalyticaly active materials preferably used in the processes of the present invention can be manufactured in a facile manner. As indicated above, a combination of two process steps was found to be crucial:

(i) spray drying or freeze drying of the aqueous solution of metal precursor and organic carbon source, and
(ii) thermal treatment at moderate temperatures of the resulting intermediate.

Accordingly, a process comprising the following steps, for the manufacture of catalytically active material, preferably used in the processes according to the invention, is presently disclosed:

(a) providing an aqueous solution comprising metal precursor and organic carbon source,
wherein the metal precursor comprises one or a combination of more than one organic, at least partially water soluble, salts of cobalt, and
wherein the organic carbon source is one or a combination of more than one di-, tri-, or polycarboxylic acids,
(b) spray drying or freeze drying the aqueous solution of metal precursor and organic carbon source and, thus, obtaining intermediate product P,
(c) thermo-treating intermediate product P at a temperature in the range from 200 °C to 380 °C.

Each of the process steps may be performed in a batch-wise or continuous format.
As indicated above, formation of the preferred catalytically active materials employed in the processes of the present invention requires a combination of spray drying or freeze drying and suitable thermal treatment at moderate temperatures. Accordingly, it appears reasonable to assume that only material present in solution, i.e. in dissolved form in the solution provided in step (a), can be transformed into catalytically active material. However, undissolved matter in solid form may be suspended in solution provided in step (a) as long as it does not interfere with the process forming the catalytically active material. Such solids, which may, for example, originate from undissolved metal precursor or organic carbon source, may form solid diluents of the catalytically active material in the solid product obtained after step (c). Similarly, organic solvents may be dissolved or emulsified in the solution provided in step (a) as long as their presence does not interfere with the process forming the catalytically active material.
If no dopant metal is used, the metal precursor in the solution provided in step (a), is one or a combination of more than one organic, at least partially water soluble, salts of cobalt. In the present context a salt is considered as being at least partially water soluble, if at least a fraction of the salt dissolves in the aqueous solution provided in step (a) under the conditions employed in the process. As indicated by experimental results, particularly good results can be achieved, if the metal precursor in the solution provided in step (a), is one or a combination of more than one, organic salts of cobalt, whereof the amounts desired to be included into the solution are completely soluble in the aqueous solution of step (a).
If dopant metal is used, the metal precursor in the solution provided in step (a) is a combination of one or more organic, at least partially water soluble, salts of cobalt, with one or more organic, at least partially water soluble, salts of manganese and/or copper. As indicated by experimental results, particularly good results can be achieved, if the metal precursor in the solution provided in step (a), is a combination of one or more organic salts of cobalt with one or more organic salts of manganese and/or copper, whereof the amounts desired to be included into the solution are completely soluble in the aqueous solution of step (a).

Suitable organic anions of the metal precursors in the solution provided in step (a) are, e.g., acetate, carbonate, oxalate, citrate, malonate, tartrate and glutarate. If nitrogen does not need to be avoided, nitrate is another suitable anion of the metal precursors in the solution provided in step (a).

Di-, tri-, or polycarboxylic acids, alone or as part of a mixture, may be used as organic carbon sources of the aqueous solution provided in step (a), as long as they support formation of the catalytically active materials. As indicated by experimental results, particularly good results can be achieved, if malonic acid, glutaric acid, salicylic acid, citric acid or mixtures thereof are used as organic carbon source of the aqueous solution provided in step (a).

The aqueous solution provided in step (a) is spray dried or freeze dried in step (b). The product obtained therefrom is referred to as intermediate product P. Process parameters for spray drying and freeze drying can be varied over a wide range as long as the drying process is performed without interruption and the combined content of water and organic solvents exhibited by intermediate product P, is below 10 wt%. As indicated by experimental results, particularly good results can be achieved, if the aqueous solution provided in step (a) is spray dried in step (b).

Thermo-treating according to step (c) is performed under defined temperature conditions and inert gas atmosphere, e.g. nitrogen, or air. A wide range of suitable furnaces for this purpose is available commercially. As indicated by experimental results, particularly good results can be achieved, if thermo-treating is performed under inert gas atmosphere, e.g. nitrogen. Heating rates during thermo-treating should be small enough to allow homogeneous distribution of heat, i.e. typically smaller than 15 K/min. Thermo treating intermediate product P is performed at a temperature in the range from 200 °C to 380 °C. In preferred As indicated by experimental results, particularly good results can be achieved, if thermo treating intermediate product P is performed at a temperature in the range from 255 °C to 375 °C. Typically, thermo treating intermediate product P is performed for a duration of 1 to 4 hours, but thermo-treating for longer or shorter intervals of time may work as well. Heating and cooling intervals are not accounted for when determining the duration of thermo treating.

Catalytically active materials used in the processes of the present invention may be used as catalysts in unmodified form or may be transformed into catalyst bodies by shaping processes (e.g. tableting, pelletizing, extrusion, coating, 3D-printing), well known to persons of skill in the art.

Preferably, the process of the present invention, is performed under the following reaction conditions:

- Temperatures in the range of 125°C to 240°C, most preferably in the range of 170°C to 210°C,
- Total pressures in the range of 30 bar to 300 bar,
- Partial pressure of carbon monoxide, p(CO) in the range of 15 bar to 150 bar,
- Partial pressure of hydrogen, p($H_2$) in the range of 15 bar to 150 bar,
- CO/$H_2$ stoichiometric ratio in the range of 0.5 to 1.5, most preferably with a CO/$H_2$ stoichiometric ratio in the range of 0.5 to 1.1,
- Mass ratio catalyst to methanol, (mass catalyst) / (mass methanol) in the range of 1/100000 to 1/10.

The overall reaction enthalpy for conversion of methanol with CO and hydrogen to propanal is negative. Hence, reactor operation with appropriate heat dissipation and heat exchange is required. For this purpose, application of reactor types like multi tube reactors, trickle bed reactors, bubble column or slurry reactors are possible modalities. Optionally, the process can be designed based on a reactor cascade either with intermediate cooling or with different temperature setpoints for the particular reactors within the cascade. Moreover, application of a reactor cascade offers the option of intermediate removal of reaction water to increase the degree of methanol conversion and yield of propanal. Recycle of reaction intermediates and/or recycle of non-converted methanol and/or carbon monoxide are additional measures that can be applied to increase propanal yield of the process.

In a particular embodiment, the present invention relates to a process according to the invention, wherein dimethylether, acetic acid and ethanol are obtained, in addition to propanal, in the product mixture.

In another embodiment, the present invention relates to a process according to the invention, wherein at least one alkyl ester of acetic acid is obtained, in addition to propanal, in the product mixture.

In another embodiment, the present invention relates to a process according to the invention, wherein the product mixture obtained from the step of contacting methanol, CO and $H_2$ with a heterogeneous catalyst, is an intermediate product, that is further transformed into a final product in subsequent process steps.

**EXAMPLES**

**Catalyst A** was prepared according to Patent Application PCT/EP2020/074523, Example 4a by dissolving 14.4 g citric acid (puriss, Sigma Aldrich) in 75 mL of deionized water under constant stirring at room temperature. In a second beaker 14.9 g Cobalt(II)-acetate tetrahydrate ((CH$_3$COO)$_2$CO * 4 H$_2$O, Sigma Aldrich), 2.9 g Cu(II)-acetate-Monohydrate ((CH$_3$COO)$_2$Cu * H$_2$O, Alfa Aesar) and 1.5 g Mn(II)-acetate tetrahydrate (Mn(CH$_3$COO)$_2$ * 4 H$_2$O, Sigma Aldrich) were dissolved in 75 mL of deionized water under constant stirring at room temperature. The Cobalt-Copper-Manganese-solution was slowly added to the citric acid solution and stirred for another 30 min at room temperature. The resultant solution was spray dried using a conventional mini spray dryer (Büchi, Mini Spray Dryer B-290) with constant inlet temperature of 220°C, outlet temperature of 125°C and 25% pump speed. The obtained powder was split into two fractions with identical mass for the final thermo-treatment.

The catalyst was thermo-treated in a muffle furnace under nitrogen atmosphere, with a 180 min ramp to 300°C, where temperature was maintained for another 4 h followed by natural cooling down.

**Catalyst B** was prepared according to Patent Application PCT/EP2020/074523, Example 4b in a similar fashion like catalyst A up to the drying step. After drying thermo-treatment occurred by heating the dried sample up to 350°C within 180 min where temperature was maintained for 4 h followed by natural cooling down.

**Catalyst C** was prepared according to Patent Application PCT/EP2020/074523, by dissolving 14.4 g citric acid (puriss, Sigma Aldrich) in 75 mL of deionized water under constant stirring at room temperature. In a second beaker 18.7 g Cobalt(II)-acetate tetrahydrate ((CH$_3$COO)$_2$Co * 4 H$_2$O, Sigma Aldrich) was dissolved in 75 mL of deionized water under constant stirring at room temperature. The Cobalt-acetate solution was slowly added to the citric acid solution and stirred for another 30 min at room temperature. The resultant solution was spray dried using a conventional mini spray dryer (Büchi, Mini Spray Dryer B-290) with constant inlet temperature of 220°C, outlet temperature of 120°C and 20% pump speed. The obtained powder was split into two fractions with identical mass for the final thermo-treatment. The sample was thermo-treated under nitrogen atmosphere. The sample was heated up to 348°C within 180 min where temperature was maintained for 4 h followed by natural cool down.

**Catalyst D** was prepared according to Patent Application PCT/EP2020/074523, by dissolving 19.4 g citric acid (puriss, Sigma Aldrich) in 100 mL of deionized water under constant stirring at room temperature. In a second beaker 19.9 g Cobalt(II)-acetate tetrahydrate ((CH$_3$COO)$_2$Co * 4 H$_2$O, Sigma Aldrich) and 3.9 g Cu(II)-acetate-Monohydrate ((CH$_3$COO)$_2$Cu * H$_2$O, Alfa Aesar) were dissolved in 100 mL of deionized water under constant stirring at room temperature. The Cobalt-Copper-solution was slowly added to the citric acid solution and stirred for another 30 min at room temperature. The resultant solution was spray dried using a conventional mini spray dryer (Büchi, Mini Spray Dryer B-290) with constant inlet temperature of 220°C, outlet temperature of 130°C and 30% pump speed. The obtained powder was thermo-treated in a tubular furnace under nitrogen atmosphere, with a 180 min ramp to 354°C, where temperature was maintained for another 4 h followed by natural cooling down.

**Catalyst E** is a catalyst with 3 wt% Cobalt on a conventional Vulcan XC72R Carbon support was obtained according to Westerhaus *et al.* (Westerhaus, Felix A., et al. "Heterogenized cobalt oxide catalysts for nitroarene reduction by pyrolysis of molecularly defined complexes" Nature Chemistry (2013) page 538, *table 1, entry 1).*

**Catalyst F** is a catalyst with 20 wt% Cobalt on a conventional Vulcan XC72R Carbon support was obtained according to Westerhaus *et al.* (Westerhaus, Felix A., et al. "Heterogenized cobalt oxide catalysts for nitroarene reduction by pyrolysis of molecularly defined complexes" Nature Chemistry (2013) page 538, *table 1, entry 1; with higher Co-loading).*

**Catalyst G** was prepared according to Patent Application PCT/EP2020/074527, by dissolving 14.4 g citric acid (puriss, Sigma Aldrich) in 75 mL of deionized water under constant stirring at room temperature. In a second beaker 18.7 g Iron(II)-acetate (Fe(CH$_3$COO)$_2$, Sigma Aldrich) was dissolved in 75 mL of deionized water under constant stirring at room temperature. The Iron-acetate solution was slowly added to the citric acid solution and stirred for another 30 min at room temperature. The resultant solution was spray dried using a conventional mini spray dryer (Büchi, Mini Spray Dryer B-290) with constant inlet temperature of 220°C, outlet temperature of 120°C and 20% pump speed. The obtained powder was split into two fractions with identical mass for the final thermo-treatment. The second sample was thermo-treated in a similar fashion under nitrogen atmosphere. The sample was heated up to 350°C within 180 min where temperature was maintained for 4 h followed by natural cool down.

**Catalyst H** was prepared according to Patent Application PCT/EP2020/074536, by dissolving 14.4 g citric acid (puriss, Sigma Aldrich) in 75 mL of deionized water under constant stirring at room temperature. In a second beaker 18.7 g Nickel(II)-acetate tetrahydrate (Ni(CH$_3$COO)$_2$ * 4 H$_2$O, Sigma Aldrich) was dissolved in 75 mL of deionized water under constant stirring at room temperature. The Nickel-acetate solution was slowly added to the citric acid solution and stirred for another 30 min at room temperature. The resultant solution was spray dried using a conventional mini spray dryer (Büchi, Mini Spray Dryer B-290) with constant inlet temperature of 220°C, outlet temperature of 120°C and 20% pump speed. The obtained powder was split into two fractions with identical mass for the final thermo-treatment. The second sample was thermo-treated in a similar fashion under nitrogen atmosphere. The sample was heated up to 350°C within 180 min where temperature was maintained for 4 h followed by natural cool down.

The materials exhibit the following characteristics which were determined by XRF (X-ray fluorescence) and TGZ analysis using a calibrated Hitachi H-7500 field transmission electron microscope operated at 100 keV, equipped with a CCD-Camera:

| ID | $d_p$ | $\omega$ | D |
|---|---|---|---|
| A | 4,5 nm | 0,51 | 11 nm |
| B | 5,0 nm | 0,58 | 10 nm |
| C | 3,5 nm | 0,59 | 6 nm |
| D | 5,0 nm | 0,65 | 9 nm |
| E | 30 nm | 0,03 | n.d.* |
| F | 55 nm | 0,20 | n.d.* |
| G | 12 nm | 0,61 | 14 nm |
| H | 11 nm | 0,58 | 12 nm |

**Testing catalytic activity**

Catalytic Reaction

Catalytic conversion of methanol was performed in 100ml Büchi reactor with a blade agitator. Heating occurred with thermostat system "limbo 350" from Büchi. Gas dosage ($N_2$, CO and $H_2$) was realized via a controllable valve connecting pressurized gas container with the reactor. Pressure release occurred via second valve. Experiments performed to prove the current invention proceeded as described:

1. Reactor has been filled with catalyst using mass between 200 mg to 1 g
2. 50 ml of methanol were added.
3. Reactor was closed.
4. Leak test was done by dosing 6.5 bar nitrogen, keeping pressure for xxx min and subsequent pressure release
5. Pressurization of reactor with 6.5 bar of nitrogen and subsequent pressure release was repeated for three times
6. Pressurization of reactor with 5 bars of hydrogen and subsequent pressure release was repeated for three times.
7. Hydrogen was dosed until the target hydrogen partial pressure is reached.
8. Carbon monoxide was dosed until the target pressure which is the sum of target for hydrogen partial pressure and target for CO partial pressure is reached.
9. Stirrer was started with agitation rate of 1000 RPM. During start of stirrer pressure drops by about 6 bars due to gas dispersion within the liquid phase
10. Reactor was heat up to target temperature in the range between 150°C and 240°C within 45min.
11. Temperature was kept constant for 4 hours.
12. Reactor was cooled down within 1 hour below 30°C
13. Residual pressure was recorded and afterwards slowly released by chopping the release valve to avoid sudden outgassing.
14. Reactor was opened.
15. Suspension sample was taken by using a 5 ml syringe and filtrating the liquid through a one-off filter Chromafil 0-45/25 PTFE into a sample flask.
16. 1 μL undiluted sample liquid was injected for GC analysis.

For GC analysis GC type Agilent 7890 equipped with a DB-WAX column (length 30 m, diameter 250 μm, film thickness 0.25 μm) and FID detector was used. FID detector was operated at 250°C with a hydrogen flow of 45 ml/min and flow of synthetic air of 450 ml/min. Injector temperature was 250°C. According to the GC analysis method, GC oven was heated with 10 k/min starting from 40°C with a hold time of 5 min until 200°C with a hold time of 9 min before post-heating to 250°C for 5 min.

Methanol conversion and selectivity of liquid phase products were determined based on GC analysis according to following formulas:

i.

$$Conversion(Methanol) = \left(1 - \frac{GC\ Area\ (Methanol)_{t=0} - GC\ Area\ (Methanol)_{t=4\ hrs}}{GC\ Area\ (Methanol)_{t=0}}\right) \cdot 100\%$$

ii.

$$Selectivity(Product\ i) = \left(\frac{n_i \cdot GC\ Area\ (Product\ i)_{t=4hrs}}{\sum_i^m n_i \cdot GC\ Area\ (Product\ i)_{t=4hrs}}\right) \cdot 100\%$$

With

t referring to reaction time
GC Area referring to peak area in the GC chromatogram
i = 1 for dimethylether and $n_1$ = 2,
i = 2 for dimethoxymethane and $n_2$ = 3,
i = 3 for methyl formiate and $n_3$ = 2,
i = 4 for propanal and $n_4$ = 3,
i = 5 for methyl acetate and ns = 3,
i = 6 for ethanol and $n_6$ = 2,
m = 6

Results of catalytic testing

| Example | Catalyst | T [°C] | P(CO) [bar] | P(H₂) [bar] | M(cat) [g] | X(CH₃OH) [%] (i) | S(Dimethylether) [%] (ii) | S(Dimethoxymethan) [%] (ii) | S(Methyl formiate) [%] (ii) | S(Propanal) [%] (ii) | S(Methyl acetate) [%] (ii) | S(ethanol) [%] (i) | Residual pressure after reaction and cooling below 30°C [bar] |
|---|---|---|---|---|---|---|---|---|---|---|---|---|---|
| 1 | A | 180 | 50 | 50 | 0,2 | 14 | 11,94 | 0,00 | 0,00 | 80,50 | 4,80 | 2,76 | 68 |
| 2 | A | 180 | 47 | 47 | 0.4 | 21 | 11,50 | 0,00 | 0,00 | 79,45 | 6,10 | 2,95 | 63 |
| 3 | A | 180 | 47 | 47 | 0.8 | 23 | 9,90 | 0,00 | 0,00 | 79,43 | 7,46 | 3,21 | 62 |
| 4 | A | 200 | 49 | 49 | 0.4 | 14 | 8,20 | 0,00 | 0,00 | 80,84 | 8,21 | 2,75 | 66 |
| 5 | A | 220 | 51 | 51 | 0.4 | 7 | 15,70 | 2,69 | 0,00 | 64,89 | 12,98 | 3,74 | 60 |
| 6 | A | 180 | 66 | 17 | 0.4 | 19 | 6,51 | 0,00 | 0,00 | 70,03 | 21,29 | 2,17 | 53 |
| 7 | A | 180 | 34 | 68 | 0,4 | 6 | 12,56 | 0,00 | 0,00 | 73,28 | 14,16 | 0,00 | 74 |
| 8 | B | 180 | 49 | 49 | 0,2 | 17,5 | 5,52 | 0,00 | 0,00 | 87,87 | 4,53 | 2,08 | 69 |
| 9 | C | 180 | 45 | 45 | 0,2 | 17,5 | 7,23 | 0,00 | 0,00 | 86,82 | 4,86 | 1,09 | 50 |
| 10 | D | 180 | 45 | 45 | 0,2 | 10 | 14,62 | 0,00 | 0,00 | 78,94 | 4,68 | 1,76 | 53 |
| 11 | E | 180 | 45 | 45 | 0,2 | 2 | 18,68 | 0,00 | 0,00 | 55,11 | 4,7 | 21,52 | 80 |
| 12 | F | 180 | 45 | 45 | 0,2 | 10 | 33,5 | 0,00 | 0,00 | 43,51 | 0,00 | 22,9 | 56 |
| 13 | G | 180 | 45 | 45 | 0,2 | 7,5 | 19,89 | 0,00 | 32,79 | 47,32 | 0,00 | 0,00 | 55 |
| 14 | H | 180 | 45 | 45 | 0,2 | 17 | 39,65 | 0,00 | 0,00 | 60,36 | 0,00 | 0,00 | 53 |

"S" designates "Selectivity"

"X" designates "Conversion"

**Claims**

1. Process for transforming methanol into a product mixture comprising propanal, comprising the following step:

   Contacting methanol, CO and $H_2$ with a heterogeneous catalyst;
   wherein the heterogeneous catalyst comprises

   - one or more transition metals selected from Co, Ni, Cu, Fe, Mn, Mo, W, Ru, Re, Rh; and
   - carbon, exhibiting a structure selected from graphitic, carbidic, aromatic or amorphous non-graphitizing.

2. Process according to claim 1, wherein the

   - one or more transition metals are selected from Co, Cu, and Mn.

3. Process according to any one of claims 1 to 2,
   wherein the heterogeneous catalyst comprises catalytically active material, comprising grains of non-graphitizing carbon with cobalt nanoparticles dispersed therein,

wherein

$d_p$, the average diameter of cobalt nanoparticles in the non-graphitizing carbon grains, is in the range of 1 nm to 20 nm,

D, the average distance between cobalt nanoparticles in the non-graphitizing carbon grains, is in the range of 2 nm to 150 nm, and

$\omega$, the combined total mass fraction of metal in the non-graphitizing carbon grains, is in the range of 30 wt% to 70 wt% of the total mass of the non-graphitizing carbon grains,

wherein $d_p$ and D are measured by TGZ-TEM as described herein,

and wherein

$d_p$, D and $\omega$ conform to the following relation:

$$4.5 \, d_p / \omega > D \geq 0.25 \, d_p / \omega.$$

4. Process according to any one of claims 1 to 3, wherein dimethylether, acetic acid and ethanol are obtained, in addition to propanal, in the product mixture.

5. Process according to any one of claims 1 to 4, wherein at least one alkyl ester of acetic acid is obtained, in addition to propanal, in the product mixture.

6. Process according to any one of claims 1 to 5, wherein the product mixture obtained from the step of contacting methanol, CO and $H_2$ with a heterogeneous catalyst, is an intermediate product, that is further transformed into a final product in subsequent process steps.

7. Process according to any one of claims 3 to 6, wherein the non-graphitizing carbon grains in the catalytically active material exhibit the following particle size distribution: d10 $\leq$ 5$\mu$m, d50 $\leq$ 40 $\mu$m, d90 $\leq$ 150 $\mu$m.

8. Process according to any one of claims 3 to 7, wherein the total mass fraction of nitrogen in the non-graphitizing carbon grains in the catalytically active material is less than 1 wt% of the total mass of the non-graphitizing carbon grains.

9. Process according to any one of claims 3 to 8, wherein $d_p$ is in the range of 1 nm to 10 nm.

10. Process according to any one of claims 3 to 9, wherein $d_p$ is in the range of 2 nm to 6 nm.

11. Process according to any one of claims 3 to 10, wherein the heterogeneous catalyst has been doped with dopant metal,

and wherein the dopant metal is selected from Mn, Cu or mixtures thereof,

and wherein the non-graphitizing carbon grains in the catalytically active material exhibit a molar ratio RDM = n(cobalt) : n(dopant metal) in the range of 2 to 15.

12. Process according to any one of claims 7 to 11, wherein in the catalytically active material, the total mass fraction of Cu is less than $10^{-4}$ wt% of the total mass of the non-graphitizing carbon grains.

13. Process according to any one of claims 1 to 12, wherein methanol, CO and $H_2$ are contacted with the heterogeneous catalyst under the following conditions:

- Temperatures in the range of 125°C to 240°C, preferably in the range of 170°C to 210°C,
- Partial pressure of carbon monoxide, p(CO), in the range of 15 bar to 150 bar,
- Partial pressure of hydrogen, p($H_2$), in the range of 15 bar to 150 bar,
- CO/$H_2$ stoichiometric ratio in the range of 0.5 to 1.5, preferably in the range of 0.5 to 1.1,
- Mass ratio of catalyst to methanol, (mass catalyst) / (mass methanol) in the range of 1/100000 to 1/10.

14. Use of a heterogeneous catalyst, for a process according to any one of claims 1 to 13.

15. Use of a heterogeneous catalyst, for a process according to claim 13.

Europäisches
Patentamt

European
Patent Office

Office européen
des brevets

**EUROPEAN SEARCH REPORT**

Application Number

EP 21 15 9856

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 4 361 707 A (HABIB MOHAMMAD M ET AL) 30 November 1982 (1982-11-30) * column 5 - column 8; claim 1; tables 2,3 * ----- | 1-15 | INV. B01J23/75 C07C45/49 C07C47/02 B01J27/20 |
| L | WO 2021/043858 A1 (EVONIK OPERATIONS GMBH [DE]) 11 March 2021 (2021-03-11) * column 17 - column 20; claim 1 * ----- | 1-15 | |

TECHNICAL FIELDS
SEARCHED (IPC)

B01J
C07C

The present search report has been drawn up for all claims

| Place of search | Date of completion of the search | Examiner |
|---|---|---|
| The Hague | 19 July 2021 | Lacombe, Céline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
............................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 15 9856

This annex lists the patent family members relating to the patent documents cited in the above-mentioned European search report.
The members are as contained in the European Patent Office EDP file on
The European Patent Office is in no way liable for these particulars which are merely given for the purpose of information.

19-07-2021

| Patent document cited in search report | | Publication date | Patent family member(s) | | Publication date |
|---|---|---|---|---|---|
| US 4361707 | A | 30-11-1982 | CA | 1186334 A | 30-04-1985 |
| | | | DE | 3228822 A1 | 24-02-1983 |
| | | | FR | 2510555 A1 | 04-02-1983 |
| | | | JP | S5826835 A | 17-02-1983 |
| | | | US | 4361707 A | 30-11-1982 |
| | | | ZA | 824921 B | 25-05-1983 |
| WO 2021043858 | A1 | 11-03-2021 | EP | 3789112 A1 | 10-03-2021 |
| | | | WO | 2021043858 A1 | 11-03-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3092211 A1, Jochen Forstner, Steffen Unser, Sarah Böhringer
- WO 2014170223 A1, Martin Köstner, Matthias Grömping, Alexander Lygin, Rudolf Burghardt

- EP 2020074523 W
- EP 2020074527 W
- EP 2020074536 W

**Non-patent literature cited in the description**

- **JENS KLABUNDE ; CHRIS BISCHOFF ; ANTHONY J. PAPA.** Propanols. In: Ullmann's Encyclopedia of Industrial Chemistry. Wiley-VCH Verlag GmbH & Co. KGaA, 25 May 2018, 5
- SUMITOMO KAGAKU. **KOICHI NAGAI ; TOSHIAKI UI.** Trends and Future of Monomer- MMA Technologies. Sumitomo Chemical Co., Ltd. Basic Chemicals Research Laboratory: R&D Report, vol. 2004-11
- **C. D. HURD ; R. N. MEINERT.** Propionaldehyde. *Organic Syntheses,* 1932, vol. 12, 64
- *Coll.,* 1943, vol. 2, 541
- **P. SABATIER ; J.-B. SANDERENS.** Dédoublement catalytique des alcools par les métaux divisés: alcools primaires forméniques. *Compt. Rend. Hebd.,* 1903, vol. 136, 921ff
- **P. SABATIER ; J.-B. SENDERENS.** Nouvelles méthodes générales d'hydrogénation. *Ann. phys. chim.,* 1905, vol. 8 (4), 398
- **WESTERHAUS, FELIX A. et al.** Heterogenized cobalt oxide catalysts for nitroarene reduction by pyrolysis of molecularly defined complexes. *Nature Chemistry,* 2013
- **B. HASSE ; J. GLÄSEL ; A.M. KERN ; D.YU. MURZIN ; B.J.M. ETZOL.** *Catalysis Today,* 01 July 2015, vol. 249, 30-37

- **EDMOND LAM ; JOHN H. T. LUONG.** Carbon Materials as Catalyst Supports and Catalysts in the Transformation of Biomass to Fuels and Chemicals. *ACS Catal.,* 2014, vol. 4, 3393-3410
- **P.W. ALBERS.** Neutron scattering study of the terminating protons in the basic structural units of non-graphitizing and graphitizing carbons. *Carbon,* 2016, vol. 109, 239-245
- **PARKER et al.** The effect of particle size, morphology and support on the formation of palladium hydride in commercial catalysts. *Chemical Science,* 2019, vol. 10, 480
- **F. ENDTER ; U. H. GEBAUER.** *Optik (Optics),* 1956, vol. 13, 97
- **K. SEIBOLD ; M. VOLL.** Distribution function for describing the particle size distribution of Soot and pyrogenic oxides. *Chemiker-Zeitung,* 1978, vol. 102 (4), 131-135
- **LOTHAR SACHS.** Statistical methods. Springer-Verlag, 1982
- **WESTERHAUS, FELIX A. et al.** Heterogenized cobalt oxide catalysts for nitroarene reduction by pyrolysis of molecularly defined complexes. *Nature Chemistry,* 2013, 538